# EUROPEAN PATENT SPECIFICATION

(11) **EP 2 885 319 B1**
(45) Date of publication and mention of the grant of the patent: **09.10.2019**
(21) Application number: 13829237.0
(22) Date of filing: 06.08.2013
(51) Int. Cl.: C07K 16/18, A61K 39/395, A61P 35/04, C07K 16/22, A61K 45/06, A61K 39/00

(54) **ANGIOPOIETIN-LIKE 4 ANTIBODY AND A METHOD OF ITS USE IN CANCER TREATMENT**
ANGIOPOIETIN-LIKE-4-ANTIKÖRPER UND VERFAHREN ZU SEINER VERWENDUNG IN DER KREBSBEHANDLUNG
ANTICORPS ANALOGUE À L'ANGIOPOÏÉTINE 4 ET SON UTILISATION DANS LE TRAITEMENT D'UN CANCER

(30) Priority: 14.08.2012 US 201261682920 P
(43) Date of publication of application: 24.06.2015
(73) Proprietor: Nanyang Technological University, Singapore 639798 (SG)
(72) Inventor: LESCAR, Julien, Singapore 639798 (SG); WONG, Yee Hwa, Singapore 639798 (SG); CHUA, Wei Min Edmond, Singapore 639798 (SG); TAN, Nguan Soon Andrew, Singapore 639798 (SG); CHONG, Han Chung Kelvin, Singapore 639798 (SG); TAN, Ming Jie, Singapore 639798 (SG); HUANG, Royston-Luke, Singapore 639798 (SG)
(74) Representative: Viering, Jentschura & Partner mbB Patent- und Rechtsanwälte
(86) International application number: PCT/SG2013/000331
(87) International publication number: WO 2014/027959

(56) References cited:
- WO-A1-2011/046515
- WO-A1-2011/046515
- WO-A1-2013/022406
- WO-A2-2006/014729
- XU Q.C. ET AL.: 'Anti-cAngptl4 Ab-conjugated N-TiO2/NaYF4:Yb,Tm nanocomposite for near infrared-triggered drug release and enhanced targeted cancer cell ablation' ADVANCED HEALTHCARE MATERIALS vol. 1, July 2012, pages 470 - 474, XP055185128
- ZHU P. ET AL.: 'Angiopoietin-like 4 protein elevates the prosurvival intracellular O2- :H202 ratio and confers anoikis resistance to tumors' CANCER CELL vol. 19, 2011, pages 401 - 415, XP028162422
- PENGCHENG ZHU ET AL: "Angiopoietin-like 4 Protein Elevates the Prosurvival Intracellular O:HORatio and Confers Anoikis Resistance to Tumors", CANCER CELL, CELL PRESS, US, vol. 19, no. 3, 4 January 2011 (2011-01-04), pages 401-415, XP028162422, ISSN: 1535-6108, DOI: 10.1016/J.CCR.2011.01.018 [retrieved on 2011-01-14]

## Description

### Field

The disclosure relates to antibodies for use in treating cancer and methods of cancer treatment.

### Background

The ANGPTL proteins belong to a superfamily of angiogenic-regulating, secreted proteins that bear the highest similarity to members of the angiopoietin family. All members of the angiopoietin-like family (Angptl 1-7) have been found in both humans and mice, except for ANGPTL5, which is a human ortholog (Grootaert et aI., 2012; Zhu et aI., 2012). The native full-length ANGPTL4 can form higher-order structures via intermolecular disulphide bonds (Yin et aI., 2009). The N-terminal region of ANGPTL4 (herein designated as nANGPTL4) is responsible for the assembly into either dimeric or tetrameric structures (Ge et aI., 2004), and oligomerisation of ANGPTL4 is important for its function as a lipolysis of triglyceride-rich lipoproteins (LPL) inhibitor (Yau et aI., 2009; Ge et aI., 2004; Yin et aI., 2009). The full-length ANGPTL4 protein undergoes proteolytic processing by proprotein convertases at the linker region, releasing the nANGPTL4 and the monomeric C-terminal portion of ANGPTL4 (cANGPTL4) (Yang et aI., 2008; Lei et aI., 2011; Ge et aI., 2004).

Angiopoietin-like protein 4 (ANGPTL4) are secreted proteins mainly expressed in liver that have been demonstrated to regulate triglyceride metabolism by inhibiting the lipolysis of triglyceride-rich lipoproteins. Experimental results show that ANGPTL4 function to regulate circulating triglyceride levels during different nutritional states and therefore play a role in lipid metabolism during feeding/fasting through differential inhibition of Lipoprotein lipase (LPL). The N-terminal domain of Angiopoietin-like proteins has been shown to play an active role in lipid metabolism. Using deletion mutants, it was demonstrated that the N-terminal domain containing fragment - (17-207) and not the C-terminal fibrinogen-like domain containing fragment - (207-460) increased the plasma triglyceride levels in mice: ANGPTL4 has been identified as a novel paracrine and, possibly, endocrine regulator of lipid metabolism and a target of peroxisome proliferators-activated receptors (PPARs). It is expressed in numerous cell types, such as adipocytes and hepatocytes, and is upregulated after fasting and hypoxia. Importantly, ANGPTL4 undergoes proteolytic processing to release its C-terminal fibrinogen-like domain (cANGPTL4), which circulates as a monomer yet whose function remains unclear. The N-terminal coiled-coil domain of ANGPTL4 (nANGPTL4) mediates the oligomerization of ANGPTL4 and binds to lipoprotein lipase to modulate lipoprotein metabolism mediating oligomerization and lipoprotein metabolism. In contrast, cANGPTL4 exists as a monomer, and its function still remains unknown. ANGPTL4 has been showed to play a context-dependent role in angiogenesis and vascular permeability. ANGPTL4, was a recently identified to be a matricellular protein implicated in regulation of energy metabolism and wound healing .

ANGPTL4 has been identified as one of the genes that can predict breast cancer to lung metastasis with the greatest frequency (Minn et aI., 2005), and further studies indicated that TGFI3 primes breast tumours for the seeding of lung metastasis through ANGPTL4 by modulating endothelial integrity to mediate lung metastasis seeding (Zhang et aI., 2008). ANGPTL4 has been identified as a prominent gene in a compact *in vivo* hypoxia signature that predicts a poor outcome in multiple tumour types (Hu et aI., 2009; Murata et aI., 2009). ANGPTL4 mRNA has been found to be up-regulated in the perinecrotic areas of many human tumours, clear-cell renal carcinomas, oral tongue squamous cell carcinomas and human gastric cancers (Nakayama et al., 2011; Le Jan et al., 2003). Tissue array analysis revealed an elevated ANGPTL4 expression in up to 40 known human epithelial tumour types, and its expression increased as tumours progressed from benign to metastatic states (Zhu et aI., 2011). TGFI3-induced ANGPTL4 enhances the retention of cancer cells in the lungs, disrupts vascular endothelial cell-cell junctions, increases the permeability of the lung capillaries, and facilitates the trans-endothelial passage of tumour cells, thus promoting the vital steps of metastasis (Padua et al., 2008). The elevated expression of ANGPTL4 in many cancers implicated a role of ANGPTL4 in tumour growth, and it was suggested to play a role in metastasis through lymphovascular invasion (Shibata et aI., 2010). Very recent data released via the Broad-Novartis Cancer Cell Line Encyclopedia further confirmed that the expression of Angptl4 was elevated in about 1000 cancer cell lines (http://www.broadinstitute.org/ccle/home).This website contains information on analysis and visualization of DNA copy number, mRNA expression and mutation data for about 1000 cell lines is available for public access.

Tumor-derived Angptl4 directly interacts with betal integrin, and hijacked intergin-mediated signalling to maintain an elevated oncogenic O;:H₂O₂ ratio. This action stimulates the redox-mediated activation of the Src machinery and, therefore, activates the downstream PI3K1PKBa and ERK signalling cascade to promote cell survival and tumour growth. Of note, the suppression of ANGPTL4 by RNAi modulates intracellular reactive oxygen species (ROS) generation to attenuate tumour growth that is associated with enhanced apoptosis in vitro and in vivo. An anti-ANGPTL4 antibody, which targets the N-terminus of the mouse ANGPTL4 protein has been reported (Desai et aI., 2007), while it was effective *in vitro* it was shown to affect the mitochondrial activities and glucose regulations of this protein *in vivo.* The inventors further demonstrated that tumour-derived cANGPTL4 instigated the disruption of endothelial continuity by directly interacting with three novel binding partners: integrin α5β1, VE-cadherin and claudin-5, in a temporally sequential manner, thus facilitating metastasis (Huang et al., 2011). WO 2011/046515 A1 discloses antibodies and siRNAs against the ANGPTL4 protein that are capable of reducing cell proliferation.

All the inhibitors of ANGPTL4 used so far have been in a laboratory setting and are not stable or suitable for industrial scale production. A specific inhibitors of ANGPTL4 that has anti-tumour properties, low immunogenicity, and can stabily survive in a patient is needed for cancer treatment.

### Summary

A first aspect of the invention includes an antibody that binds the C-terminal fibrinogen-like domain of angiopoietin like 4 protein (cANGPTL4), said antibody comprising a heavy chain and a light chain, the heavy chain comprising a V_{H} CDR1, a V_{H} CDR2, and a V_{H} CDR3, and the light chain comprising a V_{L} CDR1, a V_{L} CDR2, and a V_{L} CDR3, wherein:
said V_{L} CDR1 comprises or consists of the amino acid sequence set forth in SEQ ID NO:3;
said V_{L} CDR2 comprises or consists of the amino acid sequence set forth in SEQ ID NO:4;
said V_{L} CDR3 comprises or consists of the amino acid sequence set forth in SEQ ID NO:5;
said V_{H} CDR1 comprises or consists of the amino acid sequence set forth in SEQ ID NO:6;
said V_{H} CDR2 comprises or consists of the amino acid sequence set forth in SEQ ID NO:7;
and said V_{H} CDR3 comprises or consists of the amino acid sequence set forth in SEQ ID NO:8.

Another aspect of the invention relates to an in vitro method of reducing cell proliferation, which comprises contacting proliferating cells with an antibody as described herein.

Another aspect of the invention relates to a nucleic acid molecule encoding a heavy or light chain of the antibody

Another aspect of the invention relates to a composition for treating cancer comprising the antibody as described herein and a carrier.

Other aspects of the invention would be apparent to a person skilled in the art with reference to the following drawings and discription of various non-limiting embodiments.

### Brief description of the drawings

The drawings are not necessarily drawn to scale, emphasis instead generally being placed upon illustrating the principles of various embodiments. In the following description, various embodiments of the invention are described with reference to the following drawings.

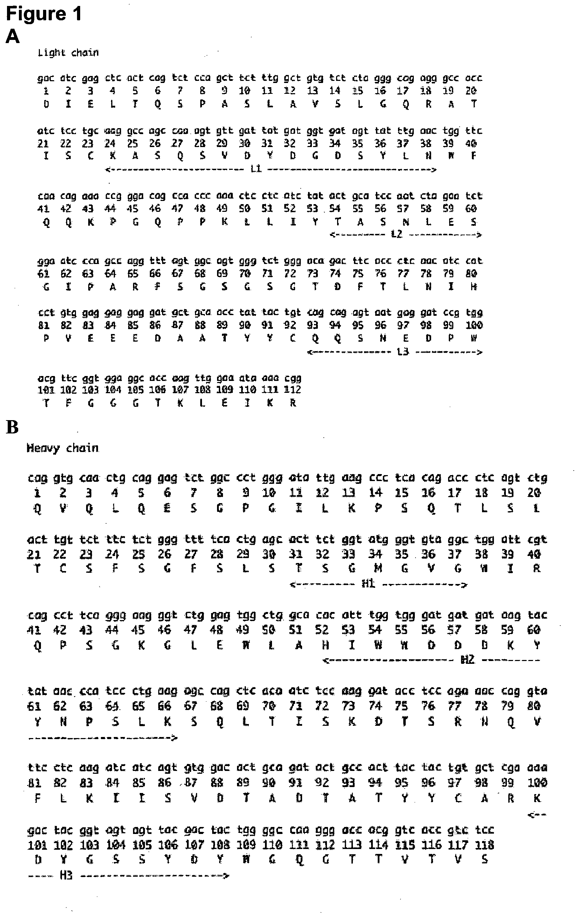

**Figure 1****:** Nucleotide (A) and amino-acid (B) sequences of mAb 11 F6C4

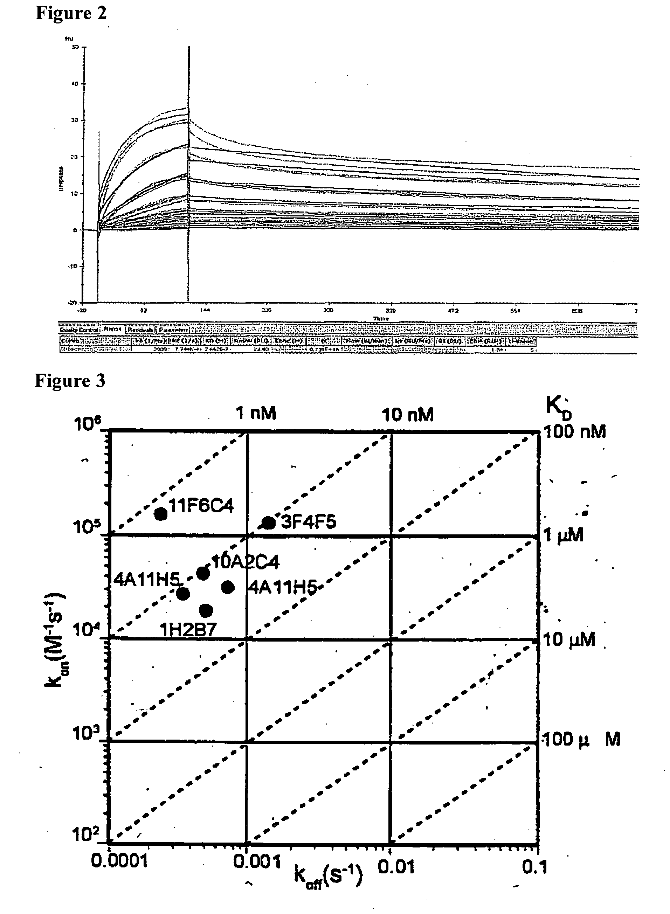

**Figure 2****:** Binding of scFV 11 F6C4 lo ANGPTL4 ANGPTL4 expressed in E. coli was attached to the chip while various dilutions of scFv 11 F6C4 were passed.
**Figure 3****:** ANGPTL4 interaction kinetic maps for human monoclonal antibodies (mAbs), shown as association and dissociation rate constant (Kon and Koff), and a combination of Kon and Koff that results in the same affinity constant (KD) values (diagonal lines) as determined by surface plasmon resonance (SPR). Labels in maps identify the many mAb clones. mAb11F6C4 was chosen for subsequent immunotherapy experiment based on its superior Kon, Koff and KD value.

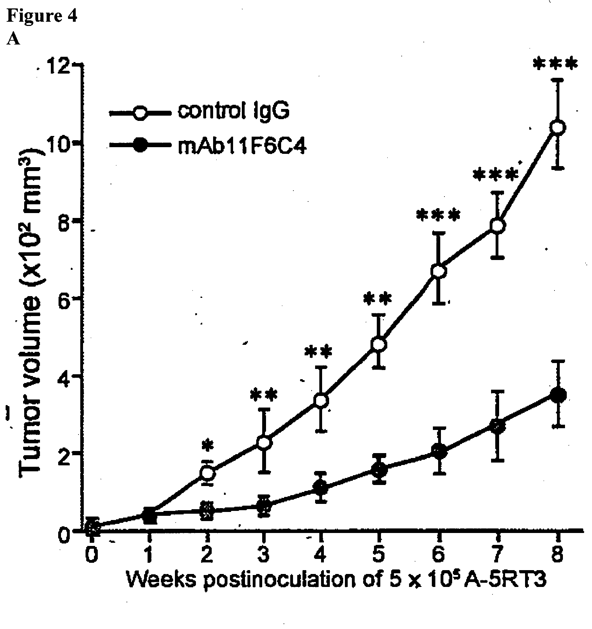

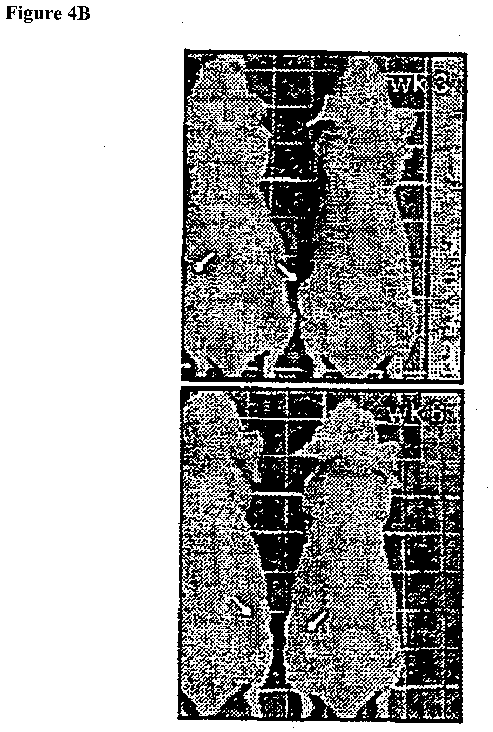

**Figure 4****:** ANGPTL4 antibody Impairs Tumorigenicity. (A) Tumor volume in nude mice injected s.c. with recombinant cells overexpressing cANGPTL4 and treated i.v. with 30 mg/kg/week of either mAb1 1F6C4 or control IgG as a function of time (n = 6 for each group). Each circle represents mean ± SEM from three measurements on each mouse. *p < 0.05; **p<0.01 ; ***p<0.001. (B) Representative pictures of control IgG- or mAb1 1F6C4-treated nude mice (wk 8) as described in (A). White arrows indicate inoculation sites.

### Detailed Description

Of particular interest is Angptl4 and its impact on tumorigenesis and metastasis. The inventors showed that mAb11 F6C4 treated mice showed reduced tumor vascular permeability, impair tumor angiogenesis and thus the mice had attenuated lung metastasis. Intriguingly, treatment with a monoclonal antibody (mAb11F6C4) against human cANGPTL4 significantly retards melanoma growth in a mouse model, reproducing the RNAi effects. Unlike another reported anti-ANGPTL4 antibody (mAb14D12), which targets the N-terminus of the mouse ANGPTL4 protein (Desai et aI., 2007), mAb11 F6C4 targets an epitope that resides within the C-terminus of human ANGPTL4, and it does not affect the mitochondrial activities and glucose regulations of this protein.

The antibody described herein is unique. The antibody 11 F6C4 and its hypervariable regions or CDRs listed in the sequence set forth in SEQ ID NO:1 and SEQ ID NO:2. is specific to the C-terminus of human ANGPTL4 suitable for cancer immunotherapy. The inventors developed stably transfected cell clones producing high-level of humanized or chimerized mAbs against Angplt4. The High-level antibody-producing clones are suitable for industrial-scale production. Such a modified mAbs, while preserving its Angptl4-binding specificities, its anti-tumor and antimetastatic properties, have been shown to possess attributes essential to enhanced clinical utility, i.e.decreased immunogenicity and longer serum half-life in patients.

Accordingly, a first aspect of the invention includes an antibody that binds the C-terminal fibrinogen-like domain of angiopoietin like 4 protein (cANGPTL4), said antibody comprising a heavy chain and a light chain, the heavy chain comprising a V_{H} CDR1, a V_{H} CDR2, and a V_{H} CDR3, and the light chain comprising a V_{L} CDR1, a V_{L} CDR2, and a V_{L} CDR3, wherein: said V_{L} CDR1 comprises or consists of the amino acid sequence set forth in SEQ ID NO:3; said V_{L} CDR2 comprises or consists of the amino acid sequence set forth in SEQ ID NO:4; said V_{L} CDR3 comprises or consists of the amino acid sequence set forth in SEQ ID NO:5; said V_{H} CDR1 comprises or consists of the amino acid sequence set forth in SEQ ID NO:6; said V_{H} CDR2 comprises or consists of the amino acid sequence set forth in SEQ ID NO:7; and said V_{H} CDR3 comprises or consists of the amino acid sequence set forth in SEQ ID NO:8.

In various embodiments the heavy chain may comprise a V_{H} domain comprising or consisting of the amino acid sequence set forth in SEQ ID NO:1 and the light chain may comprise a V_{L} domain comprising or consisting of the amino acid sequence set forth in SEQ ID NO:2.

By "antibody" herein is meant a protein consisting of one or more polypeptides substantially encoded by all or part of the recognized immunoglobulin genes. The recognized immunoglobulin genes, for example in humans, include the kappa (κ), lambda (λ), and heavy chain genetic loci, which together comprise the myriad variable region genes, and the constant region genes mu (µ), delta (δ), gamma (γ), epsilon (ε), and alpha (α) which encode the IgM, IgD, IgG (IgG1 , IgG2, IgG3, and IgG4), IgE, and IgA (IgA1 and IgA2) isotypes respectively. Antibody herein is meant to include full length antibodies and antibody fragments, and may refer to a natural antibody from any organism, an engineered antibody, or an antibody generated recombinantly for experimental, therapeutic, or other purposes.

By "IgG" as used herein is meant a polypeptide belonging to the class of antibodies that are substantially encoded by a recognized immunoglobulin gamma gene. In humans this class comprises IgG1, IgG2, IgG3, and IgG4. In mice this class comprises IgG1, IgG2a, IgG2b, IgG3.

"Specifically binding" and "specific binding", as used herein, mean that an anlibody binds to its target (analyte) based on recognition of an epitope on the target molecule. The antibody preferably recognizes and binds to the target molecule ANGPTL4 with a higher binding affinity than it binds to other compounds that may be present. In various embodiments of the invention, "specifically binding" may mean that an antibody binds to the target molecule cANGPTL4 with at least about a 10⁶-fold greater affinity, preferably at least about a 10⁷-fold greater affinity, more preferably at least about a 10⁸-fold greater affinity, and most preferably at least about a 10⁹-fold greater affinity than it binds molecules unrelated to the target molecule. Typically, specific binding refers to affinities in the range of about 10⁶-fold to about 10⁹-fold greater than non-specific binding. In some embodiments, specific binding may be characterized by affinities greater than 10⁹-fold over non-specific binding. The binding affinity may be determined by any suitable method. Such methods are known in the art and include, without limitation, surface plasmon resonance and isothermal titration calorimetry. In a specific embodiment, the antibody uniquely recognizes and binds to the target analyte.

By "immunoglobulin (Ig)" herein is meant a protein consisting of one or more polypeptides substantially encoded by immunoglobulin genes. Immunoglobulins include but are not limited to antibodies. Immunoglobulins may have a number of structural forms, including but not limited to full length antibodies, antibody fragments, and individual immunoglobulin domains. In various embodiments the immunoglobulin is an IgG1 kappa immunoglobulin.

By "immunoglobulin (Ig) domain" herein is meant a region of an immunoglobulin that exists as a distinct structural entity as ascertained by one skilled in the art of protein structure. Ig domains typically have a characteristic β-sandwich folding topology. The known Ig domains in the IgG class of antibodies are VH, Cγ1, Cy2, Cy3, VL, and CL.

By "amino acid" and "amino acid identity" as used herein is meant one of the 20 naturally occurring amino acids or any non-natural analogues that may be present at a specific, defined position. Thus "amino acid" as used herein is both naturally occurring and synthetic amino acids. For example, homophenylalanine, citrulline and noreleucine are considered amino acids for the purposes of the invention. "Amino acid" also includes imino acid residues such as proline and hydroxyproline. The side chain may be in either the (R) or the (S) configuration. In a embodiment, the amino acids are in the (S) or L-configuration. If non-naturally occurring side chains are used, non-amino acid substituents may be used, for example to prevent or retard in vivo degradation.

The variable region of an antibody contains the antigen binding determinants of the molecule, and thus determines the specificity of an antibody for its target antigen The variable region is so named because it is the most distinct in sequence from other antibodies within the same class In the variable region, three loops are gathered for each of the variable (V) domains of the heavy chain and light chain to form an antigen-binding site Each of the loops is referred to as a complementarity-determining region (hereinafter referred to as a "CDR"), in which the variation in the amino acid sequence is most significant There are 6 CDRs total, three each per heavy and light chain, designated V_{H} CDR1, V_{H} CDR2, V_{H} CDR3, V_{L} CDR1, V_{L} CDR2, and V_{L} CDR3. The variable region outside of the CDRs is referred to as the framework region (FR) Although not as diverse as the CDRs, sequence variability does occur in the FR region between different antibodies Overall, this characteristic architecture of antibodies provides a stable scaffold (the FR region) upon which substantial antigen binding diversity (the CDRs) can be explored by the immune system to obtain specificity for a broad array of antigens. A number of high-resolution structures are available for a variety of variable region fragments from different organisms, some unbound and some in complex with antigen. Sequence and structural features of antibody variable regions are disclosed, for example, in Morea et al., 1997, Biophys Chem 68:9-16; Morea et al., 2000, Methods 20:267-279, and the conserved features of antibodies are disclosed, for example, in Maynard et al., 2000, Annu Rev Biomed Eng 2:339-376.

In various embodiments the antibody comprises a heavy chain and a light chain, the heavy chain comprising a V_{H} CDR1, a V_{H} CDR2, and a V_{H} CDR3, and the light chain comprising a V_{L} CDR1, a V_{L} CDR2, and a V_{L} CDR3, wherein; said V_{L} CDR1 comprises or consists of an amino acid sequence selected from the group consisting of the amino acid sequence SEQ ID NO:3; said V_{L} CDR2 comprises or consists of an amino acid sequence selected from the group consisting of the amino acid sequence set forth in SEQ ID NO:4; said V_{L} CDR3 comprises or consists of an amino acid sequence selected from the group consisting of the amino acid sequence set forth in SEQ ID NO:5; said V_{H} CDR1 comprises or consists of an amino acid sequence selected from the group consisting of the amino acid sequence set forth in SEQ ID NO:6; said V_{H} CDR2 comprises or consists of an amino acid sequence selected from the group consisting of the amino acid sequences set forth in SEQ ID and said V_{H} CDR3 comprises or consists of an amino acid sequence selected from the group consisting of the amino acid sequence set forth in SEQ ID NO:8.

By "constant region" of an antibody as defined herein is meant the region of the antibody that is encoded by one of the light or heavy chain immunoglobulin constant region genes.

By "constant light chain" or "light chain constant region" as used herein is meant the region of an antibody encoded by the kappa (C_{κ}) or lambda (C_{λ}) light chains The constant light chain typically comprises a single domain, and as defined herein refers to positions 108-214 of C_{κ} or lambda C_{λ}, wherein numbering is according to the EU index.

By "constant heavy chain" or "heavy chain constant region" as used herein is meant the region of an antibody encoded by the mu, delta, gamma, alpha, or epsilon genes to define the antibody's isotype as IgM, IgD, IgG, IgA, or IgE, respectively For full length IgG antibodies, the constant heavy chain, as defined herein, refers to the N-terminus of the CH1 domain to the C-terminus of the CH3 domain, thus comprising positions 118-447, wherein numbering is according to the EU index.

By "variable region" as used herein is meant the region of an immunoglobulin that comprises one or more Ig domains substantially encoded by any of the Vκ, Vλ, and/or VH genes that make up the kappa, lambda, and heavy chain immunoglobulin genetic loci respectively.

By "humanized" antibody as used herein is meant an antibody comprising a human framework region (FR) and one or more complementarity determining regions (CDRs) from a non-human (usually mouse or rat) antibody. The non-human antibody providing the CDRs is called the "donor" and the human immunoglobulin providing the framework is called the "acceptor". Humanization relies principally on the grafting of donor CDRs onto acceptor (human) VL and VH frameworks (Winter, US 5,225,539). This strategy is referred to as "CDR grafting". "Backmutation" of selected acceptor framework residues to the corresponding donor residues is often required to regain affinity that is lost in the initial grafted construct (US 5,693,762). The humanized antibody optimally also will comprise at least a portion of an immunoglobulin constant region, typically that of a human immunoglobulin, and thus will typically comprise a human Fc region. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein). Humanization or other methods of reducing the immunogenicity of nonhuman antibody variable regions may include resurfacing methods, as described for example in Roguska et al., 1994, Proc Natl Acad Sci USA 91 969-973). In one embodiment, selection based methods may be employed to humanize and/or affinity mature antibody variable regions, that is, to increase the affinity of the variable region for its target antigen. Other humanization methods may involve the grafting of only parts of the CDRs, including but not limited to methods described in, Tan et al, 2002, J Immunol 169 1119-1125, De Pascalis et al, 2002, J Immunol 169 3076-3084. Structure-based methods may be employed for humanization and affinity maturation, for example as described in US patent 7,117,096 and related applications.

In various embodiments the immunoglobulin comprises a human IgG1 constant region within a heavy chain of the immunoglobulin and a human IgG1 constant region within a light chain of the immunoglobulin. In various embodiments the immunoglobulin comprises fully or partially human framework region within the variable domain of said heavy chain and within the variable domain of said light chain. In various embodiments the immunoglobulin comprises murine framework regions within the variable domain of the heavy chain and within the light chain.

Preferably the antibody described herein is suitable for use in a method for treating a tumor. A tumour as defined here is a cancer or neoplasm that may include melanoma, prostate cancer, colon cancer, liver cancer, bladder cancer, breast cancer or lung cancer. Any one of the 1000 cancer cell lines where the expression of Angptl4 was elevated is included in the term tumor or cancer. Any of the cancers from which these cancer cell lines were derived is also included in the term tumor or cancer. Metastatic cancer is included in the term tumor or cancer.

The antibodies may be monoclonal antibodies. The term "monoclonal antibody", as used herein, refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they may be synthesized by hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies can include "chimeric" antibodies (U.S. Patent No. 4,816,567; and Morrison et al. (1984) Proc. Natl. Acad. Sci. USA, 81: 6851-6855) and humanized antibodies (Jones et al. (1986) Nature, 321: 522-525; Reichmann et al. (1988) Nature, 332: 323-329; Presta (1992) Curr. Op. Struct. Biol. 2: 593-596).

Monoclonal antibodies may be obtained by any technique that provides for the production of antibody molecules by continuous cell lines in culture. These include, but are not limited to the hybridoma technique of Koehler and Milstein (1975), Nature, 256: 495-7; and U. S. Patent No. 4,376,110), the human B-cell hybridoma technique (Kosbor, et al. (1983), Immunology Today, 4: 72; Cote, et al. (1983), Proc. Natl. Acad. Sci. USA, 80: 2026-30), and the EBV-hybridoma technique (Cole, et al. (1985), in Monoclonal Antibodies And Cancer Therapy, Alan R. Liss, Inc., New York, pp. 77-96). The preparation of monoclonal antibodies specific for a target compound is also described in Harlow and Lane, eds. (1988) Antibodies - A Laboratory Manual. Cold Spring Harbor Laboratory, Chapter 6. Such antibodies may be of any immunoglobulin class including IgG, IgM, IgE, IgA, IgD and any subclass thereof. The hybridoma producing the mAb may be cultivated *in vitro* or *in vivo.* Production of high titers of mAbs *in vivo* makes this a very effective method of production.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif, and the American Type Culture Collection, Manassas, Va. Human myeloma and mouse-human heteromyeloma cell lines also have been described for the production of human monoclonal antibodies (Kozbor, J. (1984) Immunol., 133:3001).

The culture medium in which the hybridoma cells are cultured can then be assayed for the presence of monoclonal antibodies using the C-terminal fibrinogen like domain of ANGPTL4 (cANGPTL4).

The monoclonal antibodies may be made by recombinant DNA methods known in the art.

"Polyclonal antibodies" are heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

Techniques described for the production of single chain antibodies (U. S. Patent No. 4,946,778; Bird (1988), Science 242: 423-26; Huston, et al. (1988), Proc. Natl. Acad. Sci. USA, 85: 5879-83; and Ward, et al. (1989), Nature, 334: 544-46) can be adapted to produce gene-single chain antibodies. Single chain antibodies are typically formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')₂ fragments that can be produced by pepsin digestion of the antibody molecule and the Fab fragments that can be generated by reducing the disulfide bridges of the F(ab')₂ fragments. Alternatively, Fab expression libraries may be constructed (Huse, et al. (1989), Science, 246: 1275-1281) to allow rapid and easy identification of monoclonal Fab fragments with the desired specificity. The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain cross-linking. Alternatively, the relevant cysteine residues are substituted with another amino acid residue or are deleted so as to prevent cross-linking. The light chain may be SEQ ID No:1. The heavy chain may be SEQ ID No:2.

In vitro methods are also suitable for preparing monovalent antibodies. Digestion of antibodies to produce fragments thereof, particularly, Fab fragments, can be accomplished using routine techniques known in the art.

In various embodiments the variable regions comprising or consisting of the amino acid sequences set forth in SEQ ID NO:3; SEQ ID NO:4; SEQ ID NO:5; SEQ ID NO:6; SEQ ID and SEQ ID NO:8.are cloned into a Fab construct having human constant regions to produce a chimeric Fab. The chimeric Fab is stable, can be produced in industrial volume, is anti-tumour/anti-metastatic, has decreased immunogenicity and has a longer serum half-life in human serum.

The antibodies of the invention may further comprise humanized antibodies or human antibodies as described above.

Human antibodies can also be produced using various techniques known in the art, including phage display libraries. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire.

Alternatively, the antibodies can be a variety of structures, including, but not limited to antibody fragments. Antibody fragments include but are not limited to bispecific antibodies, minibodies, domain antibodies, synthetic antibodies, antibody mimetics, chimeric antibodies, antibody fusions (sometimes referred to as "antibody conjugates"), and fragments of each, respectively. Specific antibody fragments include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) the dAb fragment, which consists of a single variable region, (v) isolated CDR regions, (vi) F(ab')₂ fragments, a bivalent fragment comprising two linked Fab fragments (vii) single chain Fv molecules (scFv), wherein a VH domain and a VL domain are linked by a peptide linker which allows the two domains to associate to form an antigen binding site (viii) bispecific single chain Fv dimers and (ix) "diabodies" or "triabodies", multivalent or multispecific fragments constructed by gene fusion. The antibody fragments may be modified. For example, the molecules may be stabilized by the incorporation of disulfide bridges linking the VH and VL domains. Examples of antibody formats and architectures are described in Holliger & Hudson, 2006, Nature Biotechnology 23(9):1126-1136, and Carter 2006, Nature Reviews Immunology 6:343-357 and references cited therein.

Antibodies of the invention may include multispecific antibodies, notably bispecific antibodies, also sometimes referred to as "diabodies". These are antibodies that bind to two (or more) different antigens. Diabodies can be manufactured in a variety of ways known in the art, e.g., prepared chemically or from hybrid hybridomas. In one embodiment, the antibody is a minibody. Minibodies are minimized antibody-like proteins comprising a scFv joined to a CH3 domain. In some cases, the scFv can be joined to the Fc region, and may include some or all of the hinge region. For a description of multispecific antibodies see Holliger & Hudson, 2006, Nature Biotechnology 23(9): 1126-1136 and references cited therein.

In one embodiment, the antibody of the invention is an antibody fragment. Of particular interest are antibodies that comprise Fc regions, Fc fusions, and the constant region of the heavy chain (CH1-hinge-CH2-CH3) Antibodies of the present invention may comprise Fc fragments An Fc fragment of the present invention may comprise from 1-90% of the Fc region, e.g , 10-90%, 30-90%, etc Thus for example, an Fc fragment of the present invention may comprise an IgG1 Cγ2 domain, an IgG1 Cγ2 domain and hinge region, an IgG1 Cy3 domain, and so forth. In one embodiment, an Fc fragment of the present invention additionally comprises a fusion partner, effectively making it an Fc fragment fusion. Fc fragments may or may not contain extra polypeptide sequence.

In one embodiment, the antibodies of the invention are antibody "fusion proteins", sometimes referred to herein as "antibody conjugates". The fusion partner or conjugate partner can be proteinaceous or non-proteinaceous; the latter generally being generated using functional groups on the antibody and on the conjugate partner. Conjugate and fusion partners may be any molecule, including small molecule chemical compounds and polypeptides. For example, a variety of antibody conjugates and methods are described in Trail et al., 1999, Curr. Opin. Immunol. 11 :584-588. Possible conjugate partners include but are not limited to cytokines, cytotoxic agents, toxins, radioisotopes, chemotherapeutic agent, anti-angiogenic agents, a tyrosine kinase inhibitors, and other therapeutically active agents. In some embodiments, conjugate partners may be thought of more as payloads, that is to say that the goal of a conjugate is targeted delivery of the conjugate partner to a targeted cell, for example a cancer cell or immune cell, by the antibody. Thus, for example, the conjugation of a toxin to an antibody targets the delivery of the toxin to cells expressing the target antigen. As will be appreciated by one skilled in the art, in reality the concepts and definitions of fusion and conjugate are overlapping. The designation of an antibody as a fusion or conjugate is not meant to constrain it to any particular embodiment of the present invention. Rather, these terms are used loosely to convey the broad concept that any antibody of the present invention may be linked genetically, chemically, or otherwise, to one or more polypeptides or molecules to provide some desirable property.

Suitable conjugates include, but are not limited to, labels as described below, drugs and cytotoxic agents including, but not limited to, cytotoxic drugs (e.g., chemotherapeutic agents) or toxins or active fragments of such toxins. Suitable toxins and their corresponding fragments include diptheria A chain, exotoxin A chain, ricin A chain, abrin A chain, curcin, crotin, phenomycin, enomycin and the like. Cytotoxic agents also include radiochemicals made by conjugating radioisotopes to antibodies, or binding of a radionuclide to a chelating agent that has been covalently attached to the antibody. Additional embodiments utilize calicheamicin, aunstatins, geldanamycin, maytansine, and duocarmycins and analogs; for the latter, see U.S. patent application 2003/0050331.

In one embodiment, the antibodies of the present invention are fused or conjugated to a cytokine. By "cytokine" as used herein is meant a generic term for proteins released by one cell population that act on another cell as intercellular mediators. For example, as described in Penichet et al., 2001, J Immunol Methods 248-91-101, cytokines may be fused to antibody to provide an array of desirable properties Examples of such cytokines are lymphokines, monokines, and traditional polypeptide hormones. Included among the cytokines are growth hormone such as human growth hormone, N-methionyl human growth hormone, and bovine growth hormone; parathyroid hormone, thyroxine; insulin; proinsuhn; relaxin, prorelaxin; glycoprotein hormones such as follicle stimulating hormone (FSH), thyroid stimulating hormone (TSH), and luteinizing hormone (LH); hepatic growth factor; fibroblast growth factor, prolactin, placental lactogen; tumor necrosis factor-alpha and -beta; mulle[pi]an-inhibiting substance, mouse gonadotropin-associated peptide; inhibin; activin, vascular endothelial growth factor; integrin; thrombopoietin (TPO); nerve growth factors such as NGF-beta; platelet-growth factor, transforming growth factors (TGFs) such as TGF-alpha and TGF-beta; insulin-like growth factor-1 and -II; erythropoietin (EPO); osteoinductive factors, interferons such as interferon-alpha, beta, and -gamma; colony stimulating factors (CSFs) such as macrophage-CSF (M-CSF), granulocyte-macrophage-CSF (GM-CSF); and granulocyte-CSF (G-CSF), interleukins (ILs) such as IL-1 , IL-1alpha, IL-2, IL-3, IL-4, IL-5, IL-6, IL-7, IL-8, IL-9, IL-10, IL-11 , IL-12, IL-15, a tumor necrosis factor such as TNF-alpha or TNF-beta; C5a; and other polypeptide factors including LIF and kit ligand (KL). As used herein, the term cytokine includes proteins from natural sources or from recombinant cell culture, and biologically active equivalents of the native sequence cytokines.

In an alternate embodiment, the antibodies of the present invention are fused, conjugated, or operably linked to a toxin, including but not limited to small molecule toxins and enzymatically active toxins of bacterial, fungal, plant or animal origin, including fragments and/or variants thereof For example, a variety of immunotoxins and immunotoxin methods are described in Thrush et al., 1996, Ann. Rev. Immunol. 14:49-71. Small molecule toxins include but are not limited to calicheamicin, maytansine (US 5,208,020), trichothene, and CC1065. Dolastatin 10 analogs such as auristatin E (AE) and monomethylauristatin E (MMAE) may find use as conjugates for the antibodies of the present invention. Useful enzymatically active toxins include but are not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. The present invention further contemplates a conjugate between an antibody of the present invention and a compound with nucleolytic activity, for example a ribonuclease or DNA endonuclease such as a deoxyribonuclease (DNase).

In an alternate embodiment, an antibody of the present invention may be fused, conjugated, or operably linked to a radioisotope to form a radioconjugate. A variety of radioactive isotopes are available for the production of radioconjugate antibodies. Examples include, but are not limited to, At211, 1131, 1125, Y90, Re186, Re188, Sm153, Bi212, P32, and radioactive isotopes of Lu.

In yet another embodiment, an antibody of the present invention may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide). In an alternate embodiment, the antibody is conjugated or operably linked to an enzyme in order to employ Antibody Dependent Enzyme Mediated Prodrug Therapy (ADEPT). ADEPT may be used by conjugating or operably linking the antibody to a prodrug-activating enzyme that converts a prodrug (e.g. a peptidyl chemotherapeutic agent, see PCT application WO 81/01145) to an active anti-cancer drug. See, for example, PCT application WO 88/07378 or US patent 4,975,278. The enzyme component of the immunoconjugate useful for ADEPT includes any enzyme capable of acting on a prodrug in such a way so as to covert it into its more active, cytotoxic form. Enzymes that are useful in the method of this invention include but are not limited to alkaline phosphatase useful for converting phosphate-containing prodrugs into free drugs; arylsulfatase useful for converting sulfate-containing prodrugs into free drugs; cytosine deaminase useful for converting non-toxic 5-fluorocylosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents, carbohydrate-cleaving enzymes such as beta-galactosidase and neuramimidase useful for converting glycosylated prodrugs into free drugs, beta-lactamase useful for converting drugs derivatized with alpha-lactams into free drugs, and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, for example, Massey, 1987, Nature 328. 457-458). Antibody-abzyme conjugates can be prepared for delivery of the abzyme to a tumor cell population. A variety of additional conjugates are contemplated for the antibodies of the present invention. A variety of chemotherapeutic agents, anti-angiogenic agents, tyrosine kinase inhibitors, and other therapeutic agents are described below, which may find use as antibody conjugates.

Also disclosed herein is a method of treating a patient suffering from a disorder related to cancer, which comprises administering a therapeutically effective amount of the antibody as described herein.

"Treatment" and "treat" and synonyms thereof refer to therapeutic treatment wherein the object is to stop or reduce cell proliferation in cancerous cells. Preferably to at least affect cell proliferation will stop or halt the growth of a tumour or reduce (decrease) the size of the tumour or stop or slow metastasis. Those in need of such treatment include an individual or patient that has been diagnosed with cancer, a neoplasm or metastatic cancer. A patient or an individual refers to an animal, such as a mammal preferably a human. In various embodiments the cell is in vitro. In various other embodiments the cell is in vivo and the antibody may be administered to a subject such as a patient or individual in need of treatment.

Another aspect of the invention relates to an in vitro method of reducing cell proliferation, which comprises contacting proliferating cells with an antibody as described herein.

In various embodiments the in vitro cell is a cell line. In various other the antibody may be administered to a cell for reducing cell proliferation in cancerous cells.

Preferably the antibody administered to a subject is in a therapeutically effective amount. A therapeutically effective amount would be able to block angiopoietin like 4 protein (ANGPTL4) polypeptide in a proliferating cell in culture or at a tumour site. As used herein a "therapeutically effective amount" of the antibody will be an amount that is capable of stopping or halting cell proliferation that may result in stopping or halting growth of a tumour or reducing (decreasing) the size of the tumour. It may also refer to the prevention. Dosages and administration of an antibody of the invention in a pharmaceutical composition may be determined by one of ordinary skill in the art of clinical pharmacology or pharmacokinetics. An effective amount of the antibody to be employed therapeutically, for example an antibody as described herein, will depend, for example, upon the therapeutic objectives, the route of administration, and the condition of the mammal. Accordingly, it will be necessary for the therapist to titer the dosage and modify the route of administration as required to obtain the optimal therapeutic effect. A typical daily dosage might range from about 10 ng to up to 100 mg more per day, preferably about 1 µg to 10 mg per day. Doses may include antibody amount any where in the range of 10 to 100 µg or more preferably 25, 50, or 75 µg per day.

Another aspect of the invention relates to a nucleic acid molecule encoding a heavy or light chain of the antibody described herein.

The terms "polynucleotide" and "nucleic acid (molecule)" are used interchangeably herein to refer to polymeric forms of nucleotides of any length, including naturally occurring and non-naturally occurring nucleic acids. The polynucleotides may contain deoxyribonucleotides, ribonucleotides and/or their analogs. Methods for selection and preparation of nucleic acids are diverse and well described in standard biomolecular protocols. A typical way would be preparative PCR and chromatographic purification starting from existing template DNAs or stepwise synthesis of artificial nucleic acids. Typically, the nucleic acid molecules referred to herein are DNA molecules.

In various embodiments the nucleic acid molecule comprises a nucleotide sequence encoding the variable domain of the light chain having the nucleotide sequence set forth in SEQ ID NO:9.

Similarly, in various embodiments the nucleic acid molecule comprises a nucleotide sequence encoding the variable domain of the heavy chain set having the nucleotide sequence forth in SEQ ID NO:10.

The invention may further include an expression system comprising:
(i) a first gene encoding for a light chain; and
(ii) a second gene encoding for a heavy chain;
wherein the expression system is inducible to express an antibody as described herein.

Preferably the expression system are a cell-based expression system either in prokariotic or eukaryotic cells as described above. Preferably the expression system is a prokaryotic, heterologous expression system. In various embodiments the first gene comprises a nucleic acid sequence set forth in SEQ ID NO:9. In various other embodiments the second gene comprises a nucleic acid sequence set forth in SEQ ID NO:10.

Another aspect of the invention relates to a composition for treating cancer comprising the antibody as described herein and a carrier.

Pharmaceutical compositions are contemplated wherein an antibody of the present invention and one or more therapeutically active agents are formulated. Formulations of the antibodies of the present invention are prepared for storage by mixing the antibody having the desired degree of purity with optional pharmaceutically acceptable carriers, excipients or stabilizers (Remington's Pharmaceutical Sciences 16th edition, Osol, A. Ed., 1980), in the form of lyophilized formulations or aqueous solutions. Acceptable carriers, excipients, or stabilizers are nontoxic to recipients at the dosages and concentrations employed, and include buffers such as phosphate, citrate, acetate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; sweeteners and other flavoring agents; fillers such as microcrystalline cellulose, lactose, corn and other starches; binding agents; additives; coloring agents; salt-forming counter-ions such as sodium; metal complexes (e.g. Zn-protein complexes); and/or non-ionic surfactants such as TWEEN™, PLURONICS™ or polyethylene glycol (PEG). In one embodiment, the pharmaceutical composition that comprises the antibody of the present invention may be in a water-soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly useful are the ammonium, potassium, sodium, calcium, and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The formulations to be used for in vivo administration should be sterile. This is readily accomplished by filtration through sterile filtration membranes or other methods.

The antibodies disclosed herein may also be formulated as immunoliposomes. A liposome is a small vesicle comprising various types of lipids, phospholipids and/or surfactant that is useful for delivery of a therapeutic agent to a mammal. Liposomes containing the antibody are prepared by methods known in the art, such as described in PCT application WO 97/38731. Liposomes with enhanced circulation time are disclosed in US patent 5,013,556. The components of the liposome are commonly arranged in a bilayer formation, similar to the lipid arrangement of biological membranes. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. A chemotherapeutic agent or other therapeutically active agent is optionally contained within the liposome.

The antibody and other therapeutically active agents may also be entrapped in microcapsules prepared by methods including but not limited to coacervation techniques, interfacial polymerization (for example using hydroxymethylcellulose or gelatin-microcapsules, or poly-(methylmethacylate) microcapsules), colloidal drug delivery systems (for example, liposomes, albumin microspheres, microemulsions, nano-particles and nanocapsules), and macroemulsions. Such techniques are disclosed in Remington's Pharmaceutical Sciences 16th edition, Osol, A. Ed., 1980. Sustained-release preparations may be prepared. Suitable examples of sustained-release preparations include semipermeable matrices of solid hydrophobic polymer, which matrices are in the form of shaped articles, e.g. films, or microcapsules. Examples of sustained-release matrices include polyesters, hydrogels (for example poly(2-hydroxyethyl-methacrylate), or poly(vinylalcohol)), polylactides (US patent 3,773,919), copolymers of L-glutamic acid and gamma ethyl-L-glutamate, nondegradable ethylene-vinyl acetate, degradable lactic acid-glycolic acid copolymers such as the Lupron Depot® (which are injectable microspheres composed of lactic acid-glycolic acid copolymer and leuprolide acetate), poly-D-(-)-3-hydroxybutyric acid, and ProLease® (commercially available from Alkermes), which is a microsphere-based delivery system composed of the desired bioactive molecule incorporated into a matrix of poly-DL-lactide-co-glycolide (PLG).

Administration of the pharmaceutical composition comprising an antibody of the present invention, e.g., in the form of a sterile aqueous solution, may be done in a variety of ways, including, but not limited to orally, subcutaneously, intravenously, intranasally, intraotically, transdermally, topically (e.g., gels, salves, lotions, creams, etc.), intraperitoneally, intramuscularly, intrapulmonary, vaginally, parenterally, rectally, or intraocularly. As is known in the art, the pharmaceutical composition may be formulated accordingly depending upon the manner of introduction.

As is known in the art, protein therapeutics are often delivered by IV infusion or bolus. The antibodies of the present invention may also be delivered using such methods. For example, administration may be by intravenous infusion with 0.9% sodium chloride as an infusion vehicle or carrier.

In addition, any of a number of delivery systems are known in the art and may be used to administer the antibodies of the present invention. Examples include, but are not limited to, encapsulation in liposomes, microparticles, microspheres (eg. PLA/PGA microspheres), and the like. Alternatively, an implant of a porous, non-porous, or gelatinous material, including membranes or fibers, may be used. Sustained release systems may comprise a polymeric material or matrix such as polyesters, hydrogels, poly(vinylalcohol), polylactides, copolymers of L-glutamic acid and ethyl-L-glutamate, ethylene-vinyl acetate, lactic acid-glycolic acid copolymers such as the Lupron Depot(R), and poly-D-(-)-3-hydroxyburyric acid. It is also possible to administer a nucleic acid encoding the antibody of the current invention, for example by retroviral infection, direct injection, or coating with lipids, cell surface receptors, or other transfection agents. In all cases, controlled release systems may be used to release the antibody at or close to the desired location of action.

The dosing amounts and frequencies of administration are, in one embodiment, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

The concentration of the therapeutically active antibody in the formulation may vary from about 0.1 to 100 weight %. In one embodiment, the concentration of the antibody is in the range of 0.003 µM to 1.0 molar. In order to treat a patient, a therapeutically effective dose of the antibody of the present invention may be administered. By "therapeutically effective dose" herein is meant a dose that produces the effects for which it is administered. The exact dose will depend on the purpose of the treatment, and will be ascertainable by one skilled in the art using known techniques. Dosages may range from 0.0001 to 100 mg/kg of body weight or greater, for example 0.1, 1, 10, or 50 mg/kg of body weight, e.g., 1 to 10mg/kg of body weight.

In some embodiments, only a single dose of the antibody is used. In other embodiments, multiple doses of the antibody are administered. The elapsed time between administrations may be less than 1 hour, about 1 hour, about 1-2 hours, about 2-3 hours, about 3-4 hours, about 6 hours, about 12 hours, about 24 hours, about 48 hours, about 2-4 days, about 4-6 days, about 1 week, about 2 weeks, or more than 2 weeks.

In other embodiments the antibodies of the present invention are administered in metronomic dosing regimes, either by continuous infusion or frequent administration without extended rest periods. Such metronomic administration may involve dosing at constant intervals without rest periods. Typically such regimens encompass chronic low-dose or continuous infusion for an extended period of time, for example 1-2 days, 1-2 weeks, 1-2 months, or up to 6 months or more. The use of lower doses may minimize side effects and the need for rest periods.

In certain embodiments the antibody of the present invention and one or more other prophylactic or therapeutic agents are cyclically administered to the patient. Cycling therapy involves administration of a first agent at one time, a second agent at a second time, optionally additional agents at additional times, optionally a rest period, and then repeating this sequence of administration one or more times. The number of cycles is typically from 2-10. Cycling therapy may reduce the development of resistance to one or more agents, may minimize side effects, or may improve treatment efficacy.

The antibodies of the present invention may be administered concomitantly with one or more other therapeutic regimens or agents. The additional therapeutic regimes or agents may be used to improve the efficacy or safety of the antibody. Also, the additional therapeutic regimes or agents may be used to treat the same disease or a comorbidity rather than to alter the action of the antibody. For example, an antibody of the present invention may be administered to the patient along with chemotherapy, radiation therapy, or both chemotherapy and radiation therapy. The antibody of the present invention may be administered in combination with one or more other prophylactic or therapeutic agents, including but not limited to cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, agents that promote proliferation of hematological cells, angiogenesis inhibitors, protein tyrosine kinase (PTK) inhibitors, additional antibodies, FcyRIIb or other Fc receptor inhibitors, or other therapeutic agents.

The terms "in combination with" and "co-administration" are not limited to the administration of the prophylactic or therapeutic agents at exactly the same time. Instead, it is meant that the antibody of the present invention and the other agent or agents are administered in a sequence and within a time interval such that they may act together to provide a benefit that is increased versus treatment with only either the antibody of the present invention or the other agent or agents. In one embodiment, that the antibody and the other agent or agents act additively, in another embodiment they act synergistically. Such molecules are suitably present in combination in amounts that are effective for the purpose intended. The skilled medical practitioner can determine empirically, or by considering the pharmacokinetics and modes of action of the agents, the appropriate dose or doses of each therapeutic agent, as well as the appropriate timings and methods of administration.

In one embodiment, the antibodies of the present invention are administered with one or more additional molecules comprising antibodies or Fc. The antibodies of the present invention may be co-administered with one or more other antibodies that have efficacy in treating the same disease or an additional comorbidity, for example two antibodies may be administered that recognize two antigens that are overexpressed in a given type of cancer.

In one embodiment, the antibodies of the present invention are administered with a chemotherapeutic agent. By "chemotherapeutic agent" as used herein is meant a chemical compound useful in the treatment of cancer. Examples of chemotherapeutic agents include but are not limited to alkylating agents such as thiotepa and cyclosphosphamide; alkyl sulfonates such as busulfan, improsulfan and piposulfan, androgens such as calusterone, dromostanolone propionate, epitiostanol, mepitiostane, testolactone, anti-adrenals such as aminoglutethimide, mitotane, trilostane, anti-androgens such as flutamide, nilutamide, bicalutamide, leuprolide, and goserelin; antibiotics such as aclacinomysins, actinomycin, authramycin, azasenne, bleomycins, cactinomycin, calicheamicin, carabicin, caminomycin, carzinophilm, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, etreptozocin, tubercidin, ubenimex, zinostatin, zorubicin; anti estrogens including for example tamoxifen, raloxifene, aromatase inhibiting 4(5)-imidazoles, 4-hydroxytamoxifen, trioxifene, keoxifene, LY 117018, onapristone, and toremifene (Fareston); anti-metabolites such as methotrexate and 5-fluorouracil (5-FU); folic acid analogues such as denopterin, methotrexate, pteropterin, trimetrexate; aziridines such as benzodopa, carboquone, meturedopa, and uredopa; ethylenimines and methylamelamines including altretamine, triethylenemelamine, trietylenephosphoramide, thethylenethiophosphaoramide and trimethylolomelamine; folic acid replenisher such as frolinic acid; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; platinum analogs such as cisplatin and carboplatin; vinblastine; platinum; proteins such as arginine deiminase and asparaginase; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; taxanes, e.g. paclitaxel (TAXOL®, Bristol-Myers Squibb Oncology, Princeton, N.J.) and docetaxel (TAXOTERE®, Rhne-Poulene Rorer, Antony, Fiance); topoisomerase inhibitor RFS 2000; thymidylate synthase inhibitor (such as Tomudex); additional chemotherapeutics including aceglatone; aldophosphamide glycoside; aminolevulinic acid; amsacrine; bestrabucil; bisantrene; edatraxate; defofamine; demecolcine; diaziquone; difluoromethylornithine (DMFO); elformithine; elliptinium acetate; etoglucid; gallium nitrate; hydroxyurea; lentinan; Ionidamine; mitoguazone; mitoxantrone; mopidamol; nitracrine; pentostatin; phenamet; pirarubicin; podophyllinic acid; 2-ethylhydrazide; procarbazine; PSK®; razoxane; sizofuran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; chlorambucil; gemcitabine; 6-thioguanine; mercaptopurine; methotrexate; etoposide (VP-16); ifosfamide; mitomycin C; mitoxantrone; vincristine; vinorelbine; navelbine; novantrone; teniposide; daunomycin; aminopterin; xeloda; ibandronate; CPT-11 ;retinoic acid; esperamicins; capecitabine. Pharmaceutically acceptable salts, acids, or derivatives of any of the above may also be used.

A chemotherapeutic or other cytotoxic agent may be administered as a prodrug. By "prodrug" as used herein is meant a precursor or derivative form of a pharmaceutically active substance that is less cytotoxic to tumor cells compared to the parent drug and is capable of being enzymatically activated or converted into the more active parent form. See, for example Wilman, 1986, Biochemical Society Transactions, 615th Meeting Belfast, 14:375-382; Stella et al., "Prodrugs: A Chemical Approach to Targeted Drug Delivery," Directed Drug Delivery; and Borchardt eet al., (ed.): 247-267, Humana Press, 1985. The prodrugs that may find use with the present invention include but are not limited to phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use with the antibodies of the present invention include but are not limited to any of the aforementioned chemotherapeutic agents.

In another embodiment, the antibody is administered with one or more immunomodulatory agents. Such agents may increase or decrease production of one or more cytokines, up- or down-regulate self-antigen presentation, mask MHC antigens, or promote the proliferation, differentiation, migration, or activation state of one or more types of immune cells. Immunomodulatory agents include but are not limited to non-steroidal antiinflammatory drugs (NSAIDs) such as aspirin, ibuprofen, celecoxib, diclofenac, etodolac, fenoprofen, indomethacin, ketoralac, oxaprozin, nabumentone, sulindac, tolmentin, rofecoxib, naproxen, ketoprofen, and nabumetone, steroids (e.g. glucocorticoids, dexamethasone, cortisone, hydroxycortisone, methylprednisolone, prednisone, prednisolone, trimcinolone, azulfidineicosanoids such as prostaglandins, thromboxanes, and leukotrienes, as well as topical steroids such as anthrahn, calcipotriene, clobetasol, and tazarotene), cytokines such as TGFb, IFNα, IFNβ, IFNγ, IL-2, IL-4, IL-10, cytokine, chemokine, or receptor antagonists including antibodies, soluble receptors, and receptor-Fc fusions against BAFF, B7, CCR2, CCR5, CD2, CD3, CD4, CD6, CD7, CD8, CD11 , CD14, CD15, CD17, CD18, CD20, CD23, CD28, CD40, CD40L, CD44, CD45, CD402, CD64, CD80, CD86, CD147, CD152, complement factors (C5, D) CTLA4, eotaxin, Fas, ICAM, ICOS, IFNα, IPNβ, IFNγ, IFNAR, IgE, IL-1 , IL-2, IL-2R, IL-4, IL-5R, IL-6, IL-8, IL-9 IL-12, IL-13, IL-13R1 , IL-15, IL-18R, IL-23, integrins, LFA-1 , LFA-3, MHC, selectins, TGFβ, TNFα, TNFβ, TNF-R1, T-cell receptor, including Enbrel® (etanercept), Humira® (adalimumab), and Remicade® (infliximab), heterologous anti-lymphocyte globulin; other immunomodulatory molecules such as 2-amino-6-aryl-5 substituted pyrimidines, anti-idiotypic antibodies for MHC binding peptides and MHC fragments, azathioprine, brequinar, bromocryptine, cyclophosphamide, cyclosporine A, D-penicillamine, deoxyspergualm, FK506, glutaraldehyde, gold, hydroxychloroquine, leflunomide, malononitriloamides (e.g. leflunomide), methotrexate, minocycline, mizoribine, mycophenolate mofetil, rapamycin, and sulfasasazine.

In an alternate embodiment, antibodies of the present invention are administered with a cytokine. By "cytokine" as used herein is meant a generic term for proteins released by one cell population that act on another cell as intercellular mediators. Examples of such cytokines are lymphokines, monokines, and traditional polypeptide hormones. Included among the cytokines are growth hormones such as human growth hormone, N-methionyl human growth hormone, and bovine growth hormone; parathyroid hormone, thyroxine; insulin; proinsulin, relaxin, prorelaxin, glycoprotein hormones such as follicle stimulating hormone (FSH), thyroid stimulating hormone (TSH), and luteinizing hormone (LH), hepatic growth factor, fibroblast growth factor; prolactin, placental lactogen, tumor necrosis factor-alpha and -beta; mullerian-inhibiting substance, mouse gonadotropin-associated peptide, inhibin; activin, vascular endothelial growth factor; integrin; thrombopoietin (TPO); nerve growth factors such as NGF-beta; platelet-growth factor, transforming growth factors (TGFs) such as TGF-alpha and TGF-beta, insulin-like growth factor-1 and -II; erythropoietin (EPO), osteoinductive factors, interferons such as interferon-alpha, beta, and -gamma, colony stimulating factors (CSFs) such as macrophage-CSF (M-CSF), granulocyte-macrophage-CSF (GM-CSF), and granulocyte-CSF (G-CSF), interleukins (ILs) such as IL-1 , IL-1 alpha, IL-2, IL-3, IL-4, IL-5, IL-6, IL-7, IL-8, IL-9, IL-10, IL-11 , IL-12, IL-15, a tumor necrosis factor such as TNF-alpha or TNF-beta, and other polypeptide factors including LIF and kit ligand (KL) As used herein, the term cytokine includes proteins from natural sources or from recombinant cell culture, and biologically active equivalents of the native sequence cytokines.

In one embodiment, cytokines or other agents that stimulate cells of the immune system are co-administered with the antibody of the present invention. Such a mode of treatment may enhance desired effector function. For example, agents that stimulate NK cells, including but not limited to IL-2 may be co-administered. In another embodiment, agents that stimulate macrophages, including but not limited to C5a, formyl peptides such as N-formyl-methionyl-leucyl-phenylalanine (Beigier-Bompadre et al. (2003) Scand J. Immunol. 57. 221-8), may be co-administered Also, agents that stimulate neutrophils, including but not limited to G-CSF, GM-CSF, and the like may be administered Furthermore, agents that promote migration of such immunostimulatory cytokines may be used. Also additional agents including but not limited to interferon gamma, IL-3 and IL-7 may promote one or more effector functions.

In an alternate embodiment, cytokines or other agents that inhibit effector cell function are co-administered with the antibody of the present invention Such a mode of treatment may limit unwanted effector function

The antibodies of the present invention may be combined with other therapeutic regimens. For example, in one embodiment, the patient to be treated with an antibody of the present invention may also receive radiation therapy. Radiation therapy can be administered according to protocols commonly employed in the art and known to the skilled artisan. Such therapy includes but is not limited to cesium, iridium, iodine, or cobalt radiation. The radiation therapy may be whole body irradiation, or may be directed locally to a specific site or tissue in or on the body, such as the lung, bladder, or prostate. Typically, radiation therapy is administered in pulses over a period of time from about 1 to 2 weeks. The radiation therapy may, however, be administered over longer periods of time. For instance, radiation therapy may be administered to patients having head and neck cancer for about 6 to about 7 weeks. Optionally, the radiation therapy may be administered as a single dose or as multiple, sequential doses. The skilled medical practitioner can determine empirically the appropriate dose or doses of radiation therapy useful herein. In accordance with another embodiment of the invention, the antibody of the present invention and one or more other anti-cancer therapies are employed to treat cancer cells ex vivo. It is contemplated that such ex vivo treatment may be useful in bone marrow transplantation and particularly, autologous bone marrow transplantation. For instance, treatment of cells or tissue(s) containing cancer cells with antibody and one or more other anti-cancer therapies, such as described above, can be employed to deplete or substantially deplete the cancer cells prior to transplantation in a recipient patient.

It is of course contemplated that the antibodies of the invention may employ in combination with still other therapeutic techniques such as surgery or phototherapy.

The present disclosure is further illustrated by the following examples. However, it should be understood, that the invention is not limited to the exemplified embodiments.

### Examples of preferred embodiments

### Generation cANGPTL4 Antibodies

Recombinant ANGPTL4 proteins were purified from the conditioned medium of stable cANGPTL4-expressing S2 cells by preparative isoelectric membrane electrophoresis as previously described (Goh et al.. 2010). Rabbit polyclonal antibodies against the C-terminal region and N-terminal region of human ANGPTL4 were produced in-house and previously described (Goh et al., 2010). Monoclonal antibodies (mAbs) against human cANGPTL4 (amino acids 186-406) were made by the following; mice were immunized with adjuvant conjugated-cAngptl4. The spleen of the mouse was removed and a single cell suspension was prepared. These cells were fused with myeloma cells and cultured in hybridoma selection medium (HAT; Gibco). The fused cells were cultured in microtiter plates with peritoneal macrophages for 48 hours post-fusion (2-4x 10⁶ cells/ml). The cultures were maintained in a 5% C0₂ humidified incubator for 7-21 days, and routinely fed with HAT medium. mAbs in medium were first screened using ELISA to identify positive clones. Positive clones were expanded and recloned by a limiting dilution technique to ensure monoclonality. Next, surface plasmon resonance was performed to determine the binding kinetics of mAbs. Global fitting of the data to a Langmuir 1 : 1 model was used to determine the association (kon), dissociation (koff) and affinity constant (KD) using Scrubber2 (BioLogic Software Pte Ltd). mAb 11F6C4 was chosen for immunotherapy and other experiments based on its superior Kon, Koff and KD values as well as its ability to block interaction between cANGPTL4 and integrins.

mRNA from anti-ANGPTL-4 hybridoma cells was extracted and reversely transcribed to cDNA using SuperScript™ III First Strand Synthesis System for RT-PCR (Invitrogen). This served as the template to construct 11 F6C4 scFv using the Recombinant Phage Antibody System (RPAS, GE Healthcare). Separate antibody VH and VL-chain-encoding regions were amplified by PCR from the cDNA library using Heavy primer mix and Light primer mix respectively. Isolated, agarose gel purified VH- and VL-encoding DNA were spliced together by PCR using Linker primer mix, the primers designed to introduce a linking sequence between the two segments and specific restriction sites at both 5' (Stil) and 3' (Notl) ends of the spliced sequence. Restriction digestion with Stil and Notl endonuclease (NEB) and agarose gel purification of the digested linked product preceded ligation of this DNA into the Stil- and Notl-digested vector pEXP-Lib (Clontech). This vector was then used to transform TOP10 E.coli competent cells (Invitrogen) using a heat shock (42°C) transformation method. Transformed TOP10 E.coli was then plated on LB agar plate containing 100µg/ml Ampicillin and incubated 16 hour at 37°C. Transformed clones were screened by colony PCR and sequenced using pEXP-Lib forward primer (5'-CTGGCTTATCGAAATTAATACGACTCACT-3') and reverse primer (5'TTGGCCGCCCTAGATGCATGCTCGACCT- 3').

The predicted amino acid sequences of VH and VL were analyzed in the NCBI database using the BLAST tool, and a homology of over 90% was verified with the published data of the variable region of antibodies of the mice species Mus musculus. Three sets of primers were designed for subsequent cloning into expression vector (Table 1). First set of primers was designed to amplify the heavy chain variable domain, where the forward primer was complementary to the 5' terminal region of the chain and the reverse primer was complementary to the 3' terminal region of the chain. Second set of primers was designed to amplify the linker region (DNA fragment that encodes the [GIY₄Ser]₃, flexible linker that joins the heavy and light chain variable domains), where the forward primer was complementary to the 3' terminal of the heavy chain variable domain and the reverse primer was complementary to the 5' terminal region of the light chain variable domain. Third set of primers was designed to amplify the light chain variable domain, where the forward primer was complementary to the 5' terminal sequence of the chain and the reverse complementary to the 3' terminal sequence of chain. The amplified heavy, light and linker PCR products were gel purified and annealed together. The resulting 750bp fragment was amplified using the primers VH-forward and VL-reverse with the respective restriction sites. Pfu Turbo DNA polymerase (Invitrogen) was used in all PCR amplification reactions to obtain scFv. The Ndel- and Notl digested scFv construct was cloned into the expression vector pET28b (Novagen) digested with the same enzymes, for expression with an N-terminal 6xHistag. This pET28b-scFv was used for transformation of E. coli BL21-AI competent cells (Invitrogen).

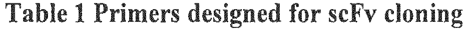

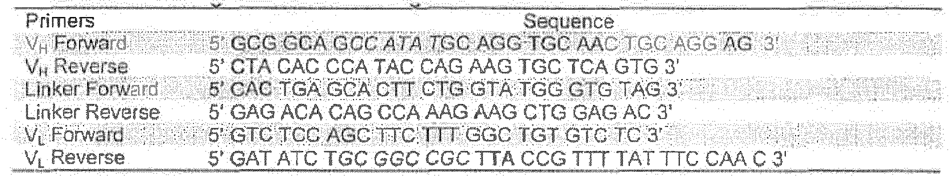

The scFv was cloned and expressed in E. coli and retains binding to a recombinant form of ANGPTL4 (Fig2).

Purified fibrinogen-like fragment of ANGPTL4 (cANGPTL4) was immobilized onto ProteOn GLC chip by amine coupling as recommended by the manufacturer (Bio-Rad). Different concentrations of integrins were introduced into the GLC chip at a flow rate of 25 µl/min for 5 min with running buffer (50mM Tris, pH8.0, 100 mM NaCl). Polyclonal anti-cANGPTL4 antibodies against the immobilized cANGPTL4 determined the Rmax value to be 423.1 resonance unit (RU). Global fitting of the data to a Languir 1 :1 model was used to determine the association (Kon) dissociation (Koff) and affinity constant (K_{D}) using scrubber2 (Biologic Software Pty Ltd). The experimental Rmax values of integrin and cANGPTL4 were determined to be 365.6 and 341.9 RU, respectively. The affinity constants of the 6 mAbs for ANGPTL4 were determined using the one shot Kinetics protocol as described by manufacturer (Bio-Rad).

To this end, the monoclonal human cANGPTL4-directed antibody mAb 11F6C4 was identified and produced for the immunotherapy experiment based on its superior Kₒₙ, K_{off} and K_{D} values, as determined by surface plasmon resonance (SPR) (Figure 3).

### In Vivo Tumorigenicity Assay

Next, the inventors reasoned that treating mice injected with recombinant cells over expressing cANGPTL4 with the antibody that interferes with the action of ANGPTL4 will stop tumour growth or reduce the tumour size.

BALB/c athymic nude female mice (20-22 g), aged 5-6 weeks, were purchased from A* STAR Biological Resources Centre (Singapore). All animals were d maintained in pathogen-free conditions. The animal studies were approved by the Institutional Animal Care and Use Committee, Nanyang Technological University, and all experiments were carried out in strict compliance with their regulations.

A total of 5x 10⁵ cells (recombinant cells overexpressing cANGPTL4) were injected subcutaneously (s.c.) into the interscapular region of each nude mouse (n = 5 for each group). Injection site was rotated to avoid site bias. The injected tumor cells were allowed to grow for eight weeks. The subcutaneous xenograft tumors were measured externally with a vernier caliper every other day, and tumor volume was estimated by using the equation, V = (L x W)/2, where L is the length of the major axis of the tumor, and W is the length of the minor axis. Mice were sacrificed at the end of the experiment (week 8), and their tumors were harvested for further analyses.

For the antibody treatment, nude mice (n = 6 for each group) were implanted with recombinant cells overexpressing cANGPTL4 as described above. One week post implantation, 30 mg/kg/week of either mAb 11F6C4 or isotype control IgG were intravenously (i. v.) administrated once weekly for four weeks. The dose of antibody and delivery mode was consistent with studies using mAb 14D12, another anti-ANGPTL4 mAb27 (Desai et al.. 2007). Mice were sacrificed after treatments and tumors were harvested for further analyses. Laser scanning microscope with a Plan-Apochromat 63x/1 .40 Oil objective and ZEN 2008 software (Carl Zeiss). Notably, immunosuppression of ANGPTL4 with mAb11F6C4 significantly attenuated in vivo tumor growth in immunodeficient mice, compared with control IgG-treated mice (n = 6 each group) (Figures 4A & 4B).

Many clinically interesting antibodies were raised in mice and prepared from hybridoma cells. The proven anti-tumor activities of these monoclonal antibodies (mAbs) in animal models have made them attractive candidates in clinical applications. However, the problem of immunogenicity of mouse mAbs in human has been a major obstacle limiting their clinical use, especially in cancer therapy, where large doses and repeated administrations are required to achieve significant efficacy. Therefore, it is desirable and essential to convert the clinically interesting mAbs of murine origin into those that are physically or functionally more human Ig like. Antibody humanization is used for reducing the immunogenicity of animal monoclonal antibodies (mAbs) and for improving their activities in the human immune system. The development of humanized monoclonal antibodies for use as human therapeutics represents one of the fastest growing segments of the biopharmaceutical industry. More and more humanized mAbs are in clinical trials and marketed as therapeutical drugs. Monoclonal antibodies is one of the current success story of the modern biotechnology R&D. Availability of human and humanized monoclonal antibodies has increased the success rate in clinical trials in cancer and immuno-inflammatory diseases like rheumatoid arthritis.

The mAb11 F6C4 disclosed herein interferes with the interaction between Angplt4 and beta1 integrin, decreases intracellular ROS production and attenuates tumor growth in vitro and in vivo. Importantly, it underscores the commercial potential of humanized mAb11 FC4 for diagnostic and therapeutic purposes aimed at metastatic cancers as well as other vascular pathologies. Monoclonal antibodies against ANGPTL4 as an anti-tumor and anti-angiogenic therapeutics.) This proposal will provide the inventors sufficient resources to develop novel redox-based approach on cancer treatment. The inventors expect new knowledge and advances in techniques to arise as a result.

By "comprising" it is meant including, but not limited to, whatever follows the word "comprising". Thus, use of the term "comprising" indicates that the listed elements are required or mandatory, but that other elements are optional and may or may not be present.

By "consisting of' is meant including, and limited to, whatever follows the phrase "consisting of'. Thus, the phrase "consisting of' indicates that the listed elements are required or mandatory, and that no other elements may be present.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

By "about" in relation to a given numberical value, such as for temperature and period of time, it is meant to include numerical values within 10% of the specified value.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non-limiting examples. In addition, where features or aspects of the invention are described in terms of Markush groups, those skilled in the art will recognize that the invention is also thereby described in terms of any individual member or subgroup of members of the Markush group.

### References

Desai, U., Lee, E.C., Chung, K., Gao, C., Gay, J., Key, B., Hansen, G., Machajewski, D., Platt, K.A., and Sands, A.T., et al. (2007). Lipid-lowering effects of anti-angiopoietin-like 4 antibody recapitulate the lipid phenotype found in angiopoietin-Iike 4 knockout mice. Proc Nat! Acad Sci USA 104,11766-11771.
Ge, H., Yang, G., Huang, L., Motola, D.L., Pourbahrami, T., and Li, C. (2004). Oligomerization and regulated proteolytic processing of angiopoietin-like protein 4. J Bioi Chern 279, 2038-2045.
Grootaert, C., Van de Wiele, T., Verstraete, W., Bracke, M., and Vanhoecke, B. (2012). Angiopoietin-like protein 4: health effects, modulating agents and structure-function relationships. Expert Rev Proteomics 9,181-199.
Hu, Z., Fan, C., Livasy, C., He, X., Oh, D.S., Ewend, M.G., Carey, L.A., Subramanian, S., West, R, and Ikpatt, F., et al. (2009). A compact VEGF signature associated with distant metastases and poor outcomes. BMC Med7, 9*.*
Lam, C.RI., Tan, M.J., Tan, S.H., Tang, M.B.Y., Cheung, P.C.F., and Tan, N.S. (2011). TAK1 regulates SCF expression to modulate PKBa activity that protects keratinocytes from ROS-induced apoptosis. Cell Death Differ 18, 1120-1129.
Le Jan, S., Amy, C., Cazes, A., Monnot, C., Lamande, N., Favier, J., Philippe, J., Sibony, M., Gasc, J.M., and Corvol, P., et al. (2003). Angiopoietin-like 4 is a proangiogenic factor produced during ischemia and in conventional renal cell carcinoma. Am J Patho/162, 1521-1528*.*
Lei, X., Shi, F., Basu, D., Huq, A., Routhier, S., Day, R, and Jin, W. (2011). Proteolytic Processing of Angiopoietin-like Protein 4 by Proprotein Convertases Modulates Its Inhibitory Effects on Lipoprotein Lipase Activity. J Biol Chem 286,15747-15756.
Minn, A.J., Gupta, G.P., Siegel, P.M., Bos, P.O., Shu, W., Giri, D.O., Via Ie, A., Olshen, A.B., Gerald, W.L., and Massague, J. (2005). Genes that mediate breast cancer metastasis to lung. Nature 436, 518-524.
Murata, M., Yudo, K., Nakamura, H., Chiba, J., Okamoto, K., Suematsu, N., Nishioka, K., Beppu, M., Inoue, K., and Kato, T., et al. (2009). Hypoxia upregulates the expression of angiopoietin-like-4 in human articular chondrocytes: role of angiopoietin-like-4 in the expression of matrix metalloproteinases and cartilage degradation. J Orthop Res 27, 50-57.
Nakayama, T., Hirakawa, H., Shibata, K., Nazneen, A., Abe, K., Nagayasu, T., and Taguchi, T. (2011). Expression of angiopoietin-like 4 (ANGPTL4) in human colorectal cancer: ANGPTL4 promotes venous invasion and distant metastasis. Oncol Rep
Padua, D., Zhang, X.H.F., Wang, Q., Nadal, C., Gerald, W.L., Gomis, R.R, and Massague, J. (2008). TGFbeta primes breast tumors for lung metastasis seeding through angiopoietin-like 4. Cell 133,66-77.
Shibata, K., Nakayama, T., Hirakawa, H., Hidaka, S., and Nagayasu, T. (2010). Clinicopathological significance of angiopoietin-like protein 4 expression in oesophageal squamous cell carcinoma. J Clin Patho/63, 1054-1058.
Wagner, B.A., Evig, C.B., Reszka, K.J., Buettner, G.R, and Burns, C.P. (2005). Doxorubicin increases intracellular hydrogen peroxide in PC3 prostate cancer cells. Arch Biochem Biophys 440, 181-190.
Yan, E.B., Unthank, J.K., Castillo-Melendez, M., Miller, S.L., Langford, S.J., and Walker, D.W. (2005). Novel method for in vivo hydroxyl radical measurement by microdialysis in fetal sheep brain in utero. J Appl Physio/98, 2304-2310*.*
Yang, Y.H., Wang, Y., Lam, K.S.L., Yau, M.H., Cheng, K.K.Y., Zhang, J., Zhu, W., Wu, D., and Xu, A. (2008). Suppression of the Raf/MEKIERK signaling cascade and inhibition of angiogenesis by the carboxyl terminus of angiopoietin-like protein 4. Arteriosc/er Thromb Vase Bioi 28, 835-840.
Yau, M.H., Wang, Y., Lam, K.S.L., Zhang, J., Wu, D., and Xu, A. (2009). A highly conserved motif within the NH2-terminal coiled-coil domain of angiopoietin-like protein 4 confers its inhibitory effects on lipoprotein lipase by disrupting the enzyme dimerization. J Bioi Chern 284,11942-11952.
Yin, W., Romeo, S., Chang, S., Grishin, N.V., Hobbs, H.H., and Cohen, J.C. (2009). Genetic variation in ANGPTL4 provides insights into protein processing and function. J Bioi Chern 284,13213-13222.
Yin, W., Romeo, S., Chang, S., Grishin, N.V., Hobbs, H.H., and Cohen, J.C. (2009). Genetic variation in ANGPTL4 provides insights into protein processing and function. J Bioi Chern 284, 13213-13222.
Zhang, Z., Cao, L., Li, J., Liang, X., Liu, Y., Liu, H., Du, J., Qu, Z., Cui, M., and Liu, S., et al. (2008). Acquisition of anoikis resistance reveals a synoikis-like survival style in BEL7402 hepatoma cells. Cancer Lett 267, 106-115.

## Claims

1. An antibody binding the C-terminal fibrinogen-like domain of angiopoietin like 4 protein (cANGPTL4), said antibody comprising a heavy chain and a light chain, the heavy chain comprising a VH CDR1, a VH CDR2, and a VH CDR3, and the light chain comprising a VL CDR1, a VL CDR2, and a VL CDR3, wherein: said VL CDR1 comprises or consists of the amino acid sequence set forth in SEQ ID NO:3; said VL CDR2 comprises or consists of the amino acid sequence set forth in SEQ ID NO:4; said VL CDR3 comprises or consists of the amino acid sequence set forth in SEQ ID NO:5; said VH CDR1 comprises or consists of the amino acid sequence set forth in SEQ ID NO:6; said VH CDR2 comprises or consists of the amino acid sequence set forth in SEQ ID NO:7; and said VH CDR3 comprises or consists of the amino acid sequence set forth in SEQ ID NO:8.

2. The antibody of claim 1, wherein the heavy chain comprises a VH domain comprising or consisting of the amino acid sequence set forth in SEQ ID NO:1 and the light chain comprises a VL domain comprising or consisting of the amino acid sequence set forth in SEQ ID NO:2.

3. The antibody of claim1 or 2, wherein the antibody is an IgG1 kappa immunoglobulin.

4. The antibody of claim 3, wherein the antibody comprises a human IgG1 constant region within a heavy chain of the immunoglobulin and a human IgG1 constant region within a light chain of the immunoglobulin.

5. The antibody of claim 3, wherein the antibody comprises a fully human framework region within the variable domain of said heavy chain and within the variable domain of said light chain.

6. The antibody of claim 3, wherein the antibody comprises murine framework regions within the variable domain of the heavy chain and within the light chain.

7. The antibody of any one of claims 1-6 for use in a method for treating a tumor.

8. An in vitro method of reducing cell proliferation, wherein the method comprises contacting proliferating cells with an antibody according to any one of claims 1-6.

9. A nucleic acid molecule encoding the antibody of any one of claims 1-6.

10. The nucleic acid molecule of claim 9, wherein the nucleic acid molecule comprises a nucleotide sequence encoding the variable domain of the light chain having the nucleotide sequence set forth in SEQ ID NO: 9.

11. The nucleic acid molecule of claim 10, wherein the nucleic acid molecule comprises a nucleotide sequence encoding the variable domain of the heavy chain having the nucleotide sequence set forth in SEQ ID NO: 10.

12. A composition for treating cancer comprising the antibody of any one of claims 1-6 and a carrier.

13. The composition of claim 12 further comprising one or more additional anti-cancer agents.

## Patentansprüche

1. Ein Antikörper, der die C-terminale Fibrinogen-ähnliche Domäne des Angiopoietin-like 4-Proteins (cANGPTL4) bindet, wobei der Antikörper eine schwere Kette und eine leichte Kette umfasst, wobei die schwere Kette eine VH CDR1, eine VH CDR2 und eine VH CDR3 umfasst, und die leichte Kette eine VL CDR1, eine VL CDR2 und eine VL CDR3 umfasst, wobei: VL CDR1 die in SEQ ID NO: 3 angegebene Aminosäuresequenz umfasst oder daraus besteht; VL CDR2 die in SEQ ID NO:4 angegebene Aminosäuresequenz umfasst oder daraus besteht; VL CDR3 die in SEQ ID NO:5 angegebene Aminosäuresequenz umfasst oder daraus besteht; VH CDR1 die in SEQ ID NO:6 angegebene Aminosäuresequenz umfasst oder daraus besteht; VH CDR2 die in SEQ ID NO:7 angegebene Aminosäuresequenz umfasst oder daraus besteht; und VH CDR3 die in SEQ ID NO:8 angegebene Aminosäuresequenz umfasst oder daraus besteht.

2. Der Antikörper gemäß Anspruch 1, wobei die schwere Kette eine VH-Domäne umfasst, die die in SEQ ID NO:1 angegebene Aminosäuresequenz umfasst oder daraus besteht und die leichte Kette eine VL-Domäne umfasst, die die in SEQ ID NO:2 angegebene Aminosäuresequenz umfasst oder daraus besteht.

3. Der Antikörper gemäß Anspruch 1 oder 2, wobei der Antikörper ein IgG1-Kappa-Immunglobulin ist.

4. Der Antikörper gemäß Anspruch 3, wobei der Antikörper eine konstante Region eines humanen IgG1 innerhalb einer schweren Kette des Immunglobulins und eine konstante Region eines humanen IgG1 innerhalb einer leichten Kette des Immunglobulins umfasst.

5. Der Antikörper gemäß Anspruch 3, wobei der Antikörper eine vollständige humane Gerüstregion innerhalb der variablen Domäne der schweren Kette und innerhalb der variablen Domäne der leichten Kette umfasst.

6. Der Antikörper gemäß Anspruch 3, wobei der Antikörper murine Gerüstregionen innerhalb der variablen Domäne der schweren Kette und innerhalb der leichten Kette umfasst.

7. Der Antikörper gemäß einem der Ansprüche 1-6 zur Verwendung in einem Verfahren zum Behandeln eines Tumors.

8. Ein In-vitro-Verfahren zum Reduzieren der Zellproliferation, wobei das Verfahren das Inkontaktbringen proliferierender Zellen mit einem Antikörper gemäß einem der Ansprüche 1-6 umfasst.

9. Ein Nukleinsäuremolekül, kodierend den Antikörper gemäß einem der Ansprüche 1-6.

10. Das Nukleinsäuremolekül gemäß Anspruch 9, wobei das Nukleinsäuremolekül eine Nukleotidsequenz umfasst, die die variable Domäne der leichten Kette, die in SEQ ID NO:9 angegebene Nukleotidsequenz aufweisend, kodiert.

11. Das Nukleinsäuremolekül gemäß Anspruch 10, wobei das Nukleinsäuremolekül eine Nukleotidsequenz umfasst, die die variable Domäne der schweren Kette, die in SEQ ID NO:10 angegebene Nukleotidsequenz aufweisend, kodiert.

12. Eine Zusammensetzung zum Behandeln von Krebs, umfassend den Antikörper gemäß einem der Ansprüche 1-6 und einen Trägerstoff.

13. Die Zusammensetzung gemäß Anspruch 12, ferner umfassend ein oder mehrere zusätzliche Antikrebsmittel.

## Revendications

1. Anticorps se liant au domaine de type fibrinogène situé à extrémité C-terminale de la protéine angiopoïétine like 4 (cANGPTL4), ledit anticorps comprenant une chaîne lourde et une chaîne légère, la chaîne lourde comprenant une CDR1 de VH, une CDR2 de VH et une CDR3 de VH, et la chaîne légère comprenant une CDR1 de VL, une CDR2 de VL et une CDR3 de VL, dans lequel : ladite CDR1 de VL comprend ou est constituée de la séquence d'acides aminés décrite dans la SEQ ID NO : 3 ; ladite CDR2 de VL comprend ou est constituée de la séquence d'acides aminés décrite dans la SEQ ID NO : 4 ; ladite CDR3 de VL comprend ou est constituée de la séquence d'acides aminés décrite dans la SEQ ID NO : 5 ; ladite CDR1 de VH comprend ou est constituée de la séquence d'acides aminés décrite dans la SEQ ID NO : 6 ; ladite CDR2 de VH comprend ou est constituée de la séquence d'acides aminés décrite dans la SEQ ID NO : 7 ; et ladite CDR3 de VH comprend ou est constituée de la séquence d'acides aminés décrite dans la SEQ ID NO : 8.

2. Anticorps selon la revendication 1, dans lequel la chaîne lourde comprend un domaine VH comprenant ou constitué de la séquence d'acides aminés décrite dans la SEQ ID NO : 1 et la chaîne légère comprend un domaine VL comprenant ou constitué de la séquence d'acides aminés décrite dans la SEQ ID NO : 2.

3. Anticorps selon la revendication 1 ou 2, dans lequel l'anticorps est l'immunoglobuline IgG1 kappa.

4. Anticorps selon la revendication 3, dans lequel l'anticorps comprend une région constante d'IgG1 humaine dans une chaîne lourde de l'immunoglobuline et une région constante d'IgG1 humaine dans une chaîne légère de l'immunoglobuline.

5. Anticorps selon la revendication 3, dans lequel l'anticorps comprend une région de charpente totalement humaine dans le domaine variable de ladite chaîne lourde et dans le domaine variable de ladite chaîne légère.

6. Anticorps selon la revendication 3, dans lequel l'anticorps comprend une région de charpente de souris dans le domaine variable de ladite chaîne lourde et dans la chaîne légère.

7. Anticorps selon l'une quelconque des revendications 1 à 6 pour une utilisation dans une méthode de traitement d'une tumeur.

8. Méthode in vitro de réduction de la prolifération cellulaire, dans laquelle la méthode comprend la mise en contact de cellules de prolifération avec un anticorps selon l'une quelconque des revendications 1 à 6.

9. Molécule d'acide nucléique codant pour l'anticorps selon l'une quelconque des revendications 1 à 6.

10. Molécule d'acide nucléique selon la revendication 9, dans laquelle la molécule d'acide nucléique comprend une séquence nucléotidique codant pour le domaine variable de la chaîne légère ayant la séquence nucléotidique décrite dans la SEQ ID NO : 9.

11. Molécule d'acide nucléique selon la revendication 10, dans laquelle la molécule d'acide nucléique comprend une séquence nucléotidique codant pour le domaine variable de la chaîne lourde ayant la séquence nucléotidique décrite dans la SEQ ID NO : 10.

12. Composition pour le traitement d'un cancer comprenant l'anticorps selon l'une quelconque des revendications 1 à 6 et un vecteur.

13. Composition selon la revendication 12 comprenant en outre un ou plusieurs agents anti-cancéreux supplémentaires.


### SEQUENCE LISTING

<110> Nanyang Technological university LESCAR, Julien WONG, Yee-Hwa CHUA, Edmond WM TAN, Andrew NS CHONG, Kelvin HC TAN, Ming-Jie HUANG, Royston-Luke
<120> Angiopoietin-like 4 Antibody and a method of its use in Cancer treatment
<130> P107324
<150> US 61/682,920
   <151> 2012-08-14
<160> 10
<170> PatentIn version 3.5
<210> 1
   <211> 112
   <212> PRT
   <213> Artificial Sequence
<220>
   <223> Antibody 11F6C4 light chain
<400> 1
   Asp Ile Glu Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Tyr Leu Asn Arg Phe Gln Gln Lys Pro Gly Gln Pro Pro
   35 40 45
   Lys Leu Leu Ile Tyr Thr Ala Ser Asn Leu Glu Ser Gly Ile Pro Ala
   50 55 60
   Arg Phe Ser Gly Ser Gly Ser Gly Thr Asp Phe Thr Leu Asn Ile His
   65 70 75 80
   Pro Val Glu Glu Glu Asp Ala Ala Thr Tyr Tyr Cys Gln Gln Ser Asn
   85 90 95
   Glu Asp Pro Trp Thr Phe Gly Gly Gly Thr Lys Leu Glu Ile Lys Arg
   100 105 110
<210> 2
   <211> 118
   <212> PRT
   <213> Artificial Sequence
<220>
   <223> Antibody 11F6C4 heavy chain
<400> 2
   Gln Val Gln Leu Gln Glu Ser Gly Pro Gly Ile Leu Lys Pro Ser Gln
   1 5 10 15
   Thr Leu Ser Leu Thr Cys Ser Phe Ser Gly Phe Ser Leu Ser Thr Ser
   20 25 30
   Gly Met Gly Val Gly Trp Ile Arg Gln Pro Ser Gly Lys Gly Leu Glu
   35 40 45
   Arg Leu Ala His Ile Trp Trp Asp Asp Asp Lys Tyr Tyr Asn Pro Ser
   50 55 60
   Leu Lys Ser Gln Leu Thr Ile Ser Lys Asp Thr Ser Arg Asn Gln Val
   65 70 75 80
   Phe Leu Lys Ile Ile Ser Val Asp Thr Ala Asp Thr Ala Thr Tyr Tyr
   85 90 95
   Cys Ala Arg Lys Asp Tyr Gly Ser Ser Tyr Asp Tyr Trp Gly Gln Gly
   100 105 110
   Thr Thr Val Thr Val Ser
   115
<210> 3
   <211> 15
   <212> PRT
   <213> Artificial sequence
<220>
   <223> VL CDR1
<400> 3
   Lys Ala Ser Gln Ser Val Asp Tyr Asp Gly Asp Ser Tyr Leu Asn
   1 5 10 15
<210> 4
   <211> 7
   <212> PRT
   <213> Artificial Sequence
<220>
   <223> VL CDR2
<400> 4
   Thr Ala Ser Asn Leu Glu Ser
   1 5
<210> 5
   <211> 8
   <212> PRT
   <213> Artificial Sequence
<220>
   <223> VL CDR3
<400> 5
   Gln Gln Ser Asn Glu Asp Pro Trp
   1 5
<210> 6
   <211> 7
   <212> PRT
   <213> Artificial Sequence
<220>
   <223> VH CDR1
<400> 6
   Thr Ser Gly Met Gly Val Gly
   1 5
<210> 7
   <211> 15
   <212> PRT
   <213> Artificial sequence
<220>
   <223> VH CDR2
<400> 7
   His Ile Trp Trp Asp Asp Asp Lys Tyr Tyr Asn Pro Ser Leu Lys
   1 5 10 15
<210> 8
   <211> 9
   <212> PRT
   <213> Artificial Sequence
<220> -
   <223> VH CDR3
<400> 8
   Lys Asp Tyr Gly Ser Ser Tyr Asp Tyr
   1 5
<210> 9
   <211> 336
   <212> DNA
   <213> Artificial Sequence
<220>
   <223> light chain
<400> 9
   gacatcgagc tcactcagtc tccagcttct ttggctgtgt ctctagggca gagggccacc    60
   atctcctgca aggccagcca aagtgttgat tatgatggtg atagttattt gaactggttc   120
   caacagaaac cgggacagcc acccaaactc ctcatctata ctgcatccaa tctagaatct   180
   ggaatcccag ccaggtttag tggcagtggg tctgggacag acttcaccct caacatccat   240
   cctgtggagg aggaggatgc tgcaacctat tactgtcagc agagtaatga ggatccgtgg   300
   acgttcggtg gaggcaccaa gttggaaata aaacgg                             336
<210> 10
   <211> 354
   <212> DNA
   <213> Artificial Sequence
<220>
   <223> heavy chain
<400> 10
   caggtacaac tgcaggagtc tggccctggg atattgaagc cctcacagac cctcagtctg    60
   acttgttctt tctctgggtt ttcactgagc acttctggta tgggtgtagg ctggattcgt   120
   cagccttcag ggaagggtct ggagtggctg gcacacattt ggtgggatga tgataagtac   180
   tataacccat ccctgaagag ccagctcaca atctccaagg atacctccag aaaccaggta   240
   ttcctcaaga tcatcagtgt ggacactgca gatactgcca cttactactg tgctcgaaaa   300
   gactacggta gtagttacga ctactggggc caagggacca cggtcaccgt ctcc         354